# FASCICULE DE BREVET EUROPEEN

(11) **EP 1 600 147 B1**
(45) Date de publication et mention de la délivrance du brevet: **02.12.2009**
(21) Numéro de dépôt: 05291029.6
(22) Date de dépôt: 13.05.2005
(51) Int. Cl.: A61K 8/34, A61K 8/41, A61K 8/42, A61K 8/44, A61K 8/46, A61Q 5/00

(54) **Utilisation de polyols particuliers pour prévenir la casse de cheveux, composition les comprenant et procédé de traitement cosmétique de cheveux**
Verwendung bestimmter Polyolen zur Verhütung brüchiger Haare, Zusammensetzung enthaltend diese Polyole, und Verfahren zur kosmetischen Verwendung an Haaren
Use of particular polyols for prevention of fragile hair, composition comprising same and process for cosmetic treatment of hair

(30) Priorité: 13.05.2004 FR 0405204
(43) Date de publication de la demande: 30.11.2005
(73) Titulaire: L'ORÉAL, 75008 Paris (FR)
(72) Inventeur: Leroy, Frédéric, 92210 Saint Cloud (FR); Philippe, Michel, 91320 Wissous (FR); Barbarat, Philippe, 92270 Bois-Colombes (FR)
(74) Mandataire: Bulle, Françoise

(56) Documents cités:
- EP-A- 0 145 618
- FR-A- 1 527 085
- FR-A- 2 604 625
- GB-A- 915 574
- GB-A- 1 179 031
- US-A- 4 035 510
- US-B1- 6 492 455
- DATABASE CAPLUS CHEMICAL ABSTRACTS SERVICE, COLUMBUS, OHIO, US; XP002308157 extrait de STN Database accession no. 1996:11413

## Description

La présente invention est relative à l'utilisation de polyols particuliers pour prévenir la casse des cheveux, en particulier des cheveux africains, à une composition cosmétique comprenant de tels polyols ainsi qu'à un procédé de traitement mettant en oeuvre cette composition.

Dans le domaine capillaire, des problèmes de casse peuvent se poser pour les cheveux, et notamment pour certains types de cheveux tels que les cheveux africains. Ces problèmes de casse se produisent généralement pendant un simple traitement mécanique comme le brossage, le peignage ou le lissage, ce traitement mécanique pouvant être effectué également pendant ou après des traitements cosmétiques comme un défrisage, un shampooing, une coloration ou une permanente. Il n'existe cependant pas aujourd'hui de traitement préventif efficace pour prévenir ou limiter ce problème que ce soit sur cheveux naturels ou traités.

Des traitements avec des solutions aqueuses d'humectant tel que la glycérine, la glycérine associée à l'urée, un poly(acide acrylique), le diméthylsulfoxyde (ou DMSO) et un poly(acide acrylique) associé au DMSO, ont été décrits dans l'article publié dans J. Soc. Cosmet. Chem, 36, Janvier/Février 1985, 39-52. Ces traitements et notamment ceux à base de poly(acide acrylique) ou de glycérine, se sont révélés être efficaces pour l'amélioration de la résistance mécanique de cheveux négroïdes et la réduction de leur taux de casse prématurée, à savoir leur taux de casse pour de faibles allongements.

GB-A-1 197 031 décrit l'utilisation en cosmétique capillaire du diméthylolurée et du diméthylolthiourée.

La Demanderesse a découvert de manière surprenante que l'utilisation de certains polyols dans le domaine capillaire, sur des cheveux naturels ou traités, permettait de prévenir leur casse et d'améliorer ainsi la résistance mécanique des cheveux.

Ces polyols sont notamment décrits dans la demande US 4 546 121 comme agents ignifugeants dans des mousses polyuréthanes et dans la demande WO 98/30627 comme agents de réticulation de polymères.

L'invention a donc pour objet l'utilisation des polyols ou de leurs sels tels que décrits ci-dessous pour prévenir la casse des cheveux.

Un autre objet de l'invention est une composition cosmétique comprenant au moins un polyol tel que décrit ci-dessous, ou sel de ce dernier.

L'invention a encore pour objet un procédé de traitement cosmétique mettant en oeuvre ladite composition.

D'autres objets, caractéristiques, aspects et avantages de l'invention apparaîtront encore plus clairement à la lecture de la description et des exemples qui suivent.

Selon l'invention, les polyols utilisés pour prévenir la casse des cheveux répondent à la formule (I) suivante : 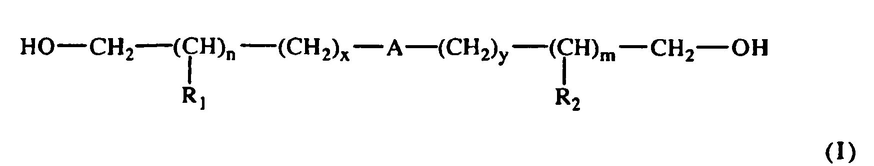 dans laquelle :
m et n représentent chacun, indépendamment l'un de l'autre, un nombre entier allant de 0 à 6, de préférence de 1 à 4,
x et y représentent chacun, indépendamment l'un de l'autre, 0 ou 1,
R₁ et R₂ représentent chacun, indépendamment l'un de l'autre, H, OH, -COOM, un atome d'halogène, un groupe alkyle en C₁-C₄ ou -NR₁'R₂' ; M représente un atome d'hydrogène, un métal alcalin ou alcalino-terreux tel que le sodium ou le potassium, ou un groupe alkyle en C₁-C₄ ;
R₁' et R₂' représentent chacun, indépendamment l'un de l'autre, H ou un groupe alkyle en C₁-C₄ ;
A représente : 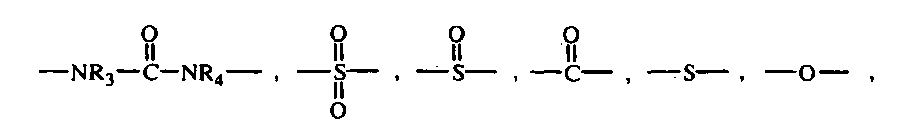 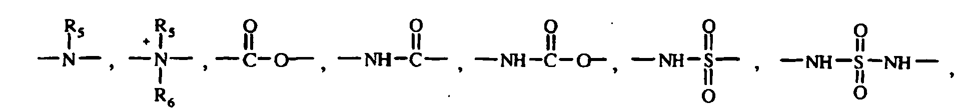 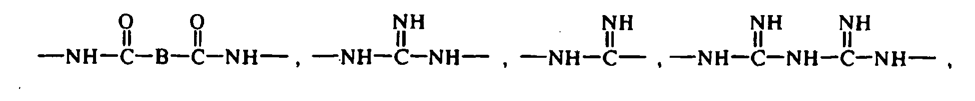
R₃, R₄, R₅ et R₆, représentent chacun, indépendamment l'un de l'autre, H ou un groupe alkyle en C₁-C₄ ;
B représente : 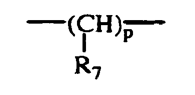
p représente un nombre entier allant de 0 à 6, de préférence de 0 à 4, et
R₇ représente H ou OH, les symboles R₇ étant identiques ou différents lorsque p est supérieur à 1.

Comme exemples de groupe alkyle en C₁-C₄, on peut notamment citer les groupes méthyle, éthyle, n-propyle, isopropyle, n-butyle ou tert-butyle.

Lorsque le polyol de formule (I) comprend des fonctions amines, amidines, guanidines et/ou bisguanidines, elles peuvent être sous la forme de sels, notamment sous forme de chlorhydrates, de sulfates ou de carboxylates d'alkyle en C₂-C₈.

Les composés préférés sont ceux de formule (I) dans laquelle :
A représente : 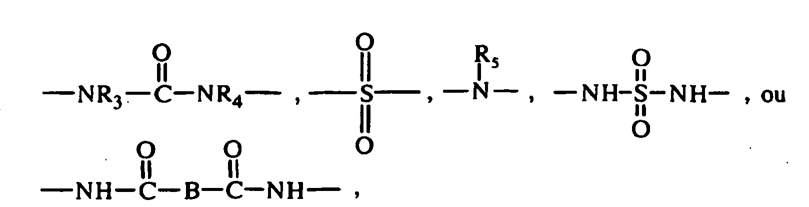
   x=y=0 ;
   R₁=R₂= R₃=R₄=H ;
   R₅= alkyle en C₁-C₄ ;
   m et n ont les mêmes significations que ci-dessus ;
B représente 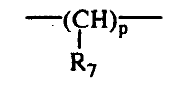 avec p = 0 à 2 et R₇ = OH.

Les polyols plus particulièrement préférés sont : 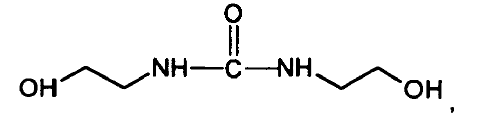 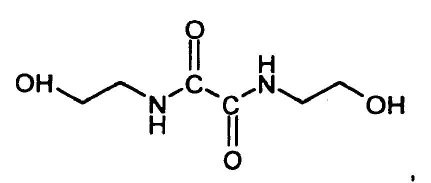 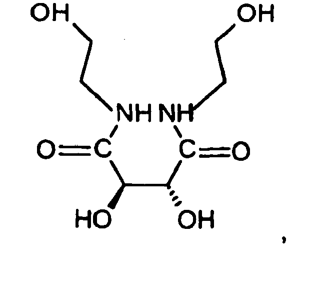 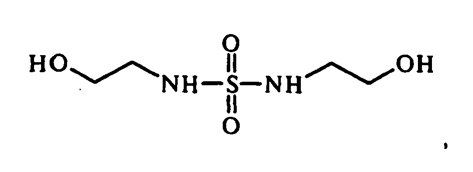 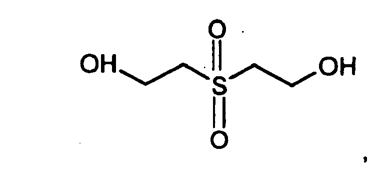 et

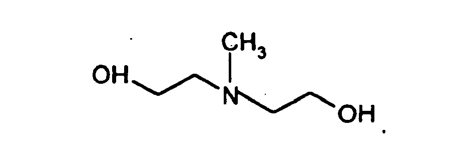

Les cheveux visés dans l'invention peuvent être d'origine asiatique, africaine ou caucasienne, et plus particulièrement d'origine africaine.

Les cheveux asiatiques sont généralement raides et pratiquement ronds.

A l'opposé, les cheveux africains présentant généralement une section assez aplatie et plus fine, forment des chevelures crépues.

Les cheveux caucasiens, quant à eux, présentent une section elliptique plus ou moins accentuée et forment ainsi des chevelures allant du raide au fortement bouclé en passant par l'ondulé.

L'invention concerne aussi une composition cosmétique comprenant, dans un milieu cosmétiquement acceptable, au moins un polyol de formule (I') suivante : 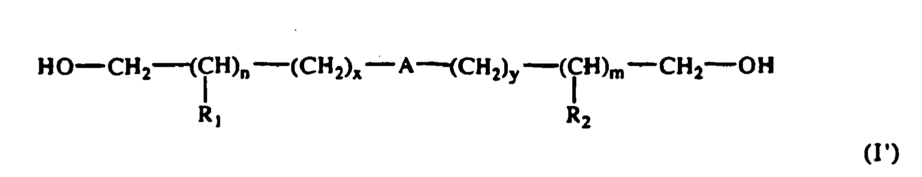 dans laquelle :
m et n représentent chacun, indépendamment l'un de l'autre, un nombre entier allant de 0 à 6, de préférence de 1 à 4,
x et y représentent chacun, indépendamment l'un de l'autre, 0 ou 1,
R₁ et R₂ représentent chacun, indépendamment l'un de l'autre, H, OH, -COOM, un atome d'halogène, un groupe alkyle en C₁-C₄ ou -NR₁'R₂' ;
M représente un atome d'hydrogène, un métal alcalin ou alcalino-terreux tel que le sodium ou le potassium, ou un groupe alkyle en C₁-C₄ ;
R₁' et R₂' représentent chacun, indépendamment l'un de l'autre, H ou un groupe alkyle en C₁-C₄ ;
A représente : 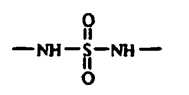 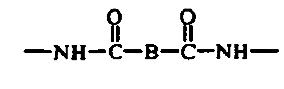
B représente : 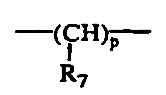
p représente un nombre entier allant de 1 à 6, de préférence de 1 à 4, et
R₇ représente H ou OH, les symboles R₇ étant identiques ou différents lorsque p est supérieur à 1, et au moins un des R₇ représentant OH, et/ou sel de ce dernier.

Lesdits sels sont notamment choisis parmi les chlorhydrates, les sulfates et les carboxylates d'alkyle en C₂-C₈.

Les polyols utilisés de préférence dans la composition selon l'invention sont ceux de formule (I') dans laquelle :
A représente : 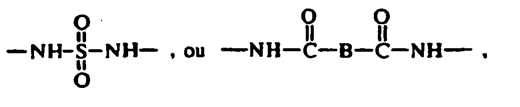
   x=y=0 ;
   R₁=R₂= H ;
B représente 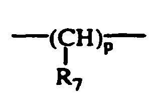 avec p = 1 ou 2 et R₇ = OH.

Des composés encore plus préférés sont choisis parmi : 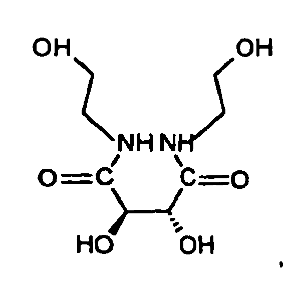 et

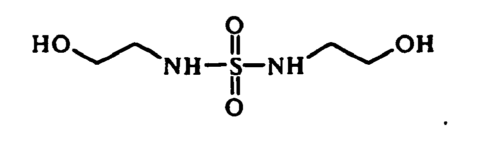

Le ou les polyols ou sels de ces derniers sont contenus de préférence en une quantité allant de 0,05 à 35 % en poids, mieux encore de 0,1 à 20 % en poids et encore plus préférentiellement de 0,5 à 10 % en poids par rapport au poids total de la composition cosmétique.

Par milieu cosmétiquement acceptable, on entend au sens de la présente invention un milieu compatible avec les cheveux.

Le milieu cosmétiquement acceptable peut être constitué uniquement par de l'eau ou par au moins un solvant organique, ou encore par un mélange d'eau et d'au moins un solvant organique. A titre de solvant organique, on peut citer, par exemple, les alcools en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol.

Les solvants sont, de préférence, présents dans des proportions de préférence comprises entre 1 et 40 % en poids environ, et encore plus préférentiellement entre 5 et 30 % en poids environ par rapport au poids total de la composition cosmétique.

Au moins un additif classiquement utilisé en cosmétique peut être également ajouté dans les compositions selon la présente invention. A titre d'exemples de tels additifs, on peut citer des agents tensioactifs anioniques, cationiques, non-ioniques, amphotères ou zwittérioniques, des polymères anioniques, cationiques, non-ioniques, amphotères ou zwittérioniques, des silicones volatiles ou non, organomodifiées ou non, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents filmogènes, des agents conservateurs, des agents opacifiants.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition cosmétique est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 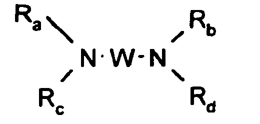 dans laquelle W est un reste propylène éventuellement substitué par un groupe hydroxyle ou alkyle en C₁-C₄ ; Rₐ, R_{b}, R_{c} et R_{d}, identiques ou différents, représentent un atome d'hydrogène, un groupe alkyle en C₁-C₄ ou hydroxyalkyle en C₁-C₄.

Les compositions cosmétiques conformes à l'invention peuvent se présenter sous forme d'une lotion, d'une crème, d'une mousse, d'un gel, d'un spray ou d'une laque et être utilisées en application rincée ou non.

Les compositions conformes à l'invention peuvent être utilisées en tant que produits capillaires, notamment rincés ou non-rincés, pour prévenir la casse des cheveux, par exemple dans un shampoing, un après-shampoing une lotion de traitement capillaire.

La présente invention concerne aussi un procédé de traitement cosmétique des cheveux, et en particulier de prévention de la casse des cheveux, comprenant l'application d'une composition cosmétique selon l'invention sur les cheveux secs ou mouillés, de préférence à une température de 10 à 60 °C, mieux encore de 20 à 40 °C, suivie d'un éventuel rinçage.

La durée du traitement peut durer de 5 minutes à 5 jours, de préférence de 10 minutes à 3 jours.

Les exemples suivants sont donnés à titre illustratif de l'invention.

### EXEMPLES

### Exemple 1 : préparation de la N,N'-bis(2-hydroxyéthyl)urée

On ajoute 100 g de 2-oxazolidinone à 70 ml d'éthanolamine. On chauffe le milieu réactionnel à 150 °C pendant 4 heures. On ajoute ensuite 200 ml de 1-butanol au milieu puis on maintient celui-ci à 0°C pendant 12 heures.

On obtient alors un précipité et on filtre, puis on le lave avec 200 ml d'heptane.

Le précipité obtenu (130 g) est ensuite repris avec 1,2 litre d'acétone sous forte agitation pendant 1 heure.

On filtre ensuite le précipité puis on le fait sécher sous vide. On obtient 70 g d'une poudre blanche (Rendement : 41%)

Les spectres RMN du ¹H et du ¹³C sont ceux de la N,N'-bis(2-hydroxyéthyl)urée. Une analyse élémentaire a donné :
C : 40,30
H : 8,04
N : 18,62 et
O : 32,87.

### Exemple 2 : préparation du 2,3-dihydroxy-N,N'-bis(2-hydroxyéthyl)-butanediamide

On ajoute 10g de (+)L- tartrate de diméthyle à une solution de 6,9 g d'éthanolamine dissous dans 10 ml de méthanol.

On chauffe le mélange réactionnel à 65°C pendant 3 heures puis on le laisse à température ambiante pendant 1 heure.

On filtre le précipité obtenu puis on le fait sécher sous pression réduite.

On fait recristalliser le produit brut obtenu dans le minimum d'éthanol bouillant. On obtient alors 8,7 g d'une poudre blanche (Rendement : 65%)

Les spectres RMN du ¹H et du ¹³C sont ceux du 2,3-dihydroxy-N,N'-bis(2-hydroxyéthyl)-butanediamide. Une analyse élémentaire a donné :
C : 40,93
H : 6,87
N : 11,73 et
O : 40,59.

### Exemple 3 : préparation du N,N'-bis(2-hydroxyéthyl)oxamide

On ajoute, goutte à goutte, une solution de 17,2 g d'éthanolamine dissous dans 20 ml de 1-hexanol, à une suspension de 20 g d'oxalate d'éthyle dans 100 ml de 1-hexanol.

On chauffe ensuite le mélange réactionnel au reflux (100 °C) pendant 2 heures puis on laisse le mélange à température ambiante pendant 1 heure.

On filtre ensuite le précipité obtenu et on le fait recristalliser dans le minimum d'éthanol bouillant. On obtient alors 20 g d'une poudre blanche (Rendement : 83%).

Les spectres RMN du ¹H et du ¹³C sont ceux du N,N'-bis(2-hydroxyéthyl)oxamide. Une analyse élémentaire a donné :
C : 40,69
H : 6,82
N : 15,72 et
O : 36,42

### Exemple 4 : préparation du N,N'-bis(2-hydroxyéthyl)sulfamide

A une solution de 0,90 g de soude (0,0225 mole) dissous dans 30 ml d'eau et 15 ml d'éthanol, on ajoute 2,36 g de sulfonyl-bis-N-oxazolidinone (0,01 mole). On maintient le milieu réactionnel à température ambiante pendant 72 heures.

On fait ensuite évaporer l'éthanol sous pression réduite puis on neutralise le milieu réactionnel à une température proche de 5°C par addition en goutte à goutte d'une solution d'acide chlorhydrique 1 N.

On soumet de nouveau le milieu réactionnel à une évaporation sous pression réduite puis on reprend avec 150 ml d'éthanol absolu.

On filtre les sels sur un verre fritté et on soumet le filtrat à une évaporation à sec.

On obtient ainsi 1,5 g de N,N'-bis(2-hydroéthyl)sulfamide sous forme d'une huile translucide (Rendement : 85%).

Les spectres RMN du ¹H, 400MHz, et RMN du ¹³C, 100 MHz, ainsi que le spectre de masse sont ceux du N,N'-bis(2-hydroxyéthyl)sulfamide.
RMN du ¹H, 400MHz, dans le DMSO d6 : δ : 2,87 ppm (t, 4H), 334 ppm (t, 4H), 5,71 ppm (se)
RMN du ¹³C, 100MHz, dans le DMSO d6 : δ : 45,14 ppm, 60,28 ppm.

### Exemple 5

On a laissé les cheveux utilisés pour les essais suivants, dans une boîte à gants à 25°C et 45 % d'humidité relative pendant environ 24 heures.

On a préparé une composition en mélangeant 5 % en poids du composé de l'exemple 1 et 95 % en poids d'eau par rapport au poids total de la composition.

On a ensuite traité 30 cheveux africains naturels avec cette composition : on a immergé les cheveux pendant 3 jours à 40 °C, sans rinçage final, puis on a comparé leur taux de casses prématurées par rapport à celui d'un lot de cheveux témoins, naturels non traités.

Des essais mécaniques ont été réalisés à l'aide d'un appareil de traction Zwick sur des cheveux de 30 mm de long. Les cheveux ont été étirés avec une vitesse de 10 mm/min.

Le taux de casses prématurées des cheveux africains, traités d'une part et témoins d'autre part, a été évalué comme suit :
R=1-(nombre de cheveux présentant une rupture sous traction dans la zone de durcissement /N)
N étant le nombre total de cheveux testés.

Les résultats sont regroupés dans le tableau suivant :

| | Premier essai | Deuxième essai |
|---|---|---|
| Cheveux traités | R = 0,17 | R = 0,10 |
| N = 30 | | |
| Cheveux témoins | R = 0,27 | R = 0,27 |
| N = 30 | | |

On observe une nette diminution du taux de casses prématurées des cheveux traités avec la composition selon l'invention.

## Revendications

1. Utilisation en cosmétique de polyols de formule (I) : 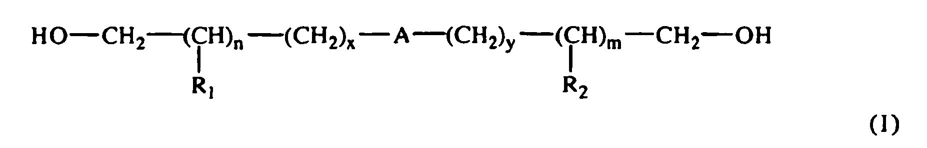 dans laquelle :
m et n représentent chacun, indépendamment l'un de l'autre, un nombre entier allant de 0 à 6,
x et y représentent chacun, indépendamment l'un de l'autre, 0 ou 1,
R₁ et R₂ représentent chacun, indépendamment l'un de l'autre, H, OH, -COOM, un atome d'halogène, un groupe alkyle en C₁-C₄ ou -NR₁'R₂' ;
M représente un atome d'hydrogène, un métal alcalin ou alcalino-terreux, ou un groupe alkyle en C₁-C₄ ;
R₁' et R₂' représentent chacun, indépendamment l'un de l'autre, H ou un groupe alkyle en C₁-C₄ ;
A représente : 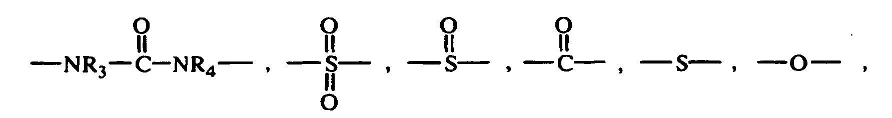 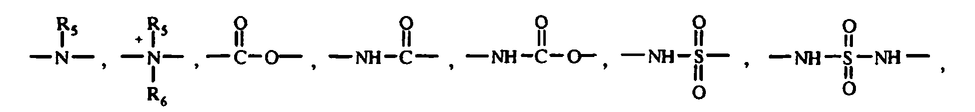 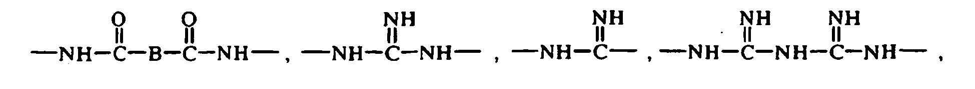
R₃, R₄, R₅ et R₆, représentent chacun, indépendamment l'un de l'autre, H ou un groupe alkyle en C₁-C₄ ;
B représente : 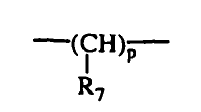
p représente un nombre entier allant de 0 à 6, et
R₇ représente H ou OH, les symboles R₇ étant identiques ou différents lorsque p est supérieur à 1,
ou de l'un de ses sels, pour prévenir la casse des cheveux.

2. Utilisation selon la revendication 1, **caractérisée en ce que**
A représente : 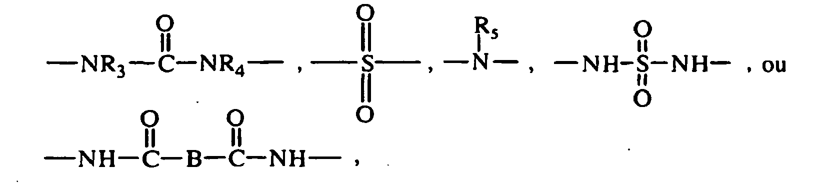
x=y=0 ;
R₁=R₂= R₃=R₄=H ;
R₅= alkyle en C₁-C₄ ;
B représente 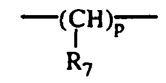 avec p = 0 à 2 et R₇ = OH.

3. Utilisation selon la revendication 1 ou 2, **caractérisée en ce que** les polyols sont choisis parmi : 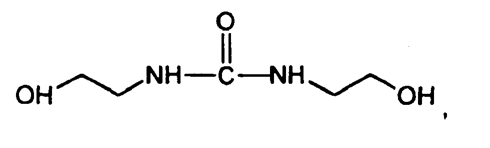 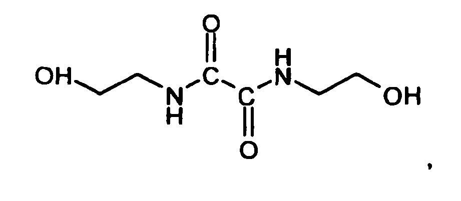 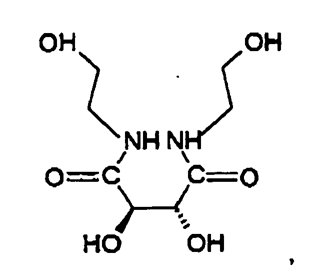 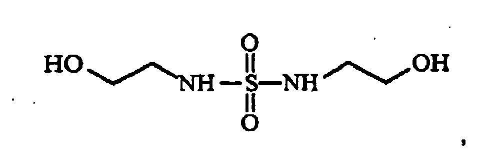 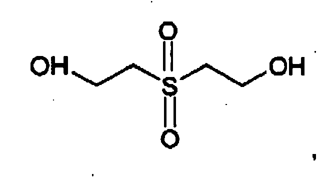 et

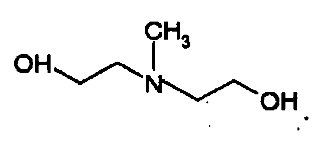

4. Utilisation selon l'une quelconque des revendications précédentes, **caractérisée en ce que** les sels sont choisis parmi les chlorhydrates, les sulfates et les carboxylates d'alkyle en C₂-C₈.

5. Utilisation selon la revendication 4, **caractérisée en ce que** les cheveux sont d'origine africaine.

6. Composition cosmétique comprenant, dans un milieu cosmétiquement acceptable, au moins un polyol répondant à la formule (I') suivante :

7. Composition cosmétique selon la revendication 6, **caractérisée en ce que**
A représente : 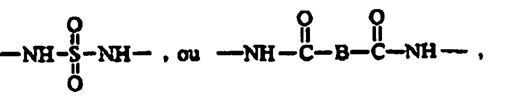
x=y=0 ;
R₁=R₂= H ;
B représente 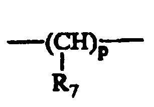 avec p = 1 ou 2 et R₇ = OH.

8. Composition selon la revendication 7, **caractérisée en ce que** le polyol est choisi parmi : 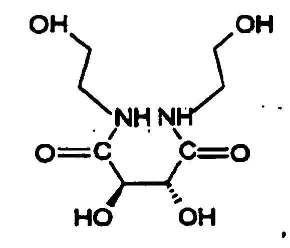 et

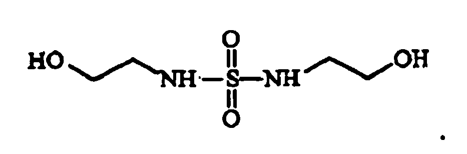

9. Composition selon l'une quelconque des revendications 6 à 8, **caractérisée en ce que** les sels sont choisis parmi les chlorhydrates, les sulfates et les carboxylates d'alkyle en C₂-C₈. 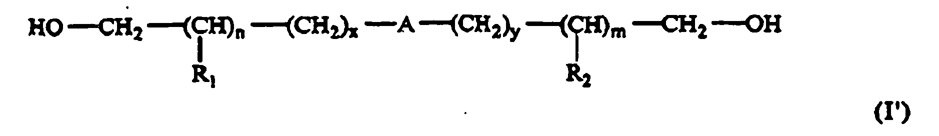 dans laquelle :
m et n représentent chacun, indépendamment l'un de l'autre, un nombre entier allant de 0 à 6,
x et y représentent chacun, indépendamment l'un de l'autre, 0 ou 1,
R₁ et R₂ représentent chacun, indépendamment l'un de l'autre, H, OH, -COOM, un atome d'halogène, un groupe alkyle en C₁-C₄ ou - NR₁'R₂' ;
M représente un atome d'hydrogène, un métal alcalin ou alcalino-terreux, ou un groupe alkyle en C₁-C₄ ;
R₁' et R₂' représentent chacun, indépendamment l'un de l'autre, H ou un groupe alkyle en C₁-C₄ ;
A représente : 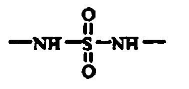 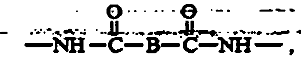
B représente : 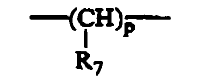
p représente un nombre entier allant de 1 à 6, et
R₇ représente H ou OH, les symboles R₇ étant identiques ou différents lorsque p est supérieur à 1, et au moins un des R₇ représentant OH, et/ou sel de ce dernier.

10. Composition cosmétique selon l'une quelconque des revendications 6 à 9, **caractérisée en ce que** le ou les polyols ou sels sont contenus en une quantité allant de 0,05 à 35 % en poids par rapport au poids total de la composition.

11. Composition cosmétique selon la revendication 10, **caractérisée en ce que** le ou les polyols ou sels sont contenus en une quantité allant de 0,1 à 20 % en poids par rapport au poids total de la composition.

12. Composition selon l'une quelconque des revendications 6 à 11, **caractérisée en ce que** le milieu cosmétiquement acceptable est constitué uniquement par de l'eau ou par au moins un solvant organique, ou encore par un mélange d'eau et d'au moins un solvant organique.

13. Composition selon la revendication 12, **caractérisée en ce que** le solvant organique est un alcool en C₁-C₄, un polyol, un éther de polyol, un alcool aromatique.

14. Composition selon la revendication 13, **caractérisée en ce que** le solvant organique est l'éthanol, l'isopropanol, le 2-butoxyéthanol, le propylèneglycol, le monométhyléther de propylèneglycol, le monoéthyléther du diéthylèneglycol, le monométhyléther du diéthylèneglycol, l'alcool benzylique ou le phénoxyéthanol.

15. Composition selon l'une quelconque des revendications 6 à 14, **caractérisée en ce que** la composition comprend au moins un additif choisi parmi les agents tensioactifs anioniques, cationiques, non-ioniques, amphotères ou zwittérioniques, les polymères anioniques, cationiques, non-ioniques, amphotères ou zwittérioniques, des silicones volatiles ou non, organomodifiées ou non, les agents épaississants minéraux ou organiques, les agents antioxydants, les agents de pénétration, les agents séquestrants, les parfums, les tampons, les agents dispersants, les agents filmogènes, les agents conservateurs et les agents opacifiants.

16. Utilisation de la composition cosmétique selon l'une quelconque des revendications 6 à 15, pour la prévention de la casse des cheveux.

17. Procédé de traitement cosmétique des cheveux, comprenant l'application d'une composition cosmétique selon l'une quelconque des revendications 6 à 15, sur les cheveux secs ou mouillés, suivie d'un éventuel rinçage.

18. Procédé de traitement cosmétique selon la revendication 17, **caractérisé en ce que** la température de traitement va de 10 à 60 °C.

19. Procédé de traitement cosmétique selon la revendication 17 ou 18, **caractérisé en ce que** la durée du traitement va de 5 minutes à 5 jours, de préférence de 10 minutes à 3 jours.

## Claims

1. Cosmetic use of polyols of formula (I): 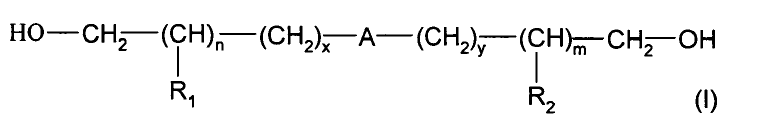 in which:
m and n each represent, independently of each other, an integer ranging from 0 to 6,
x and y each represent, independently of each other, 0 or 1,
R₁ and R₂ each represent, independently of each other, H, OH, -COOM, a halogen atom or a C₁-C₄ alkyl or -NR₁'R₂' group;
M represents a hydrogen atom, an alkali metal or alkaline-earth metal, or a C₁-C₄ alkyl group;
R₁' and R₂' each represent, independently of each other, H or a C₁-C₄ alkyl group;
A represents: 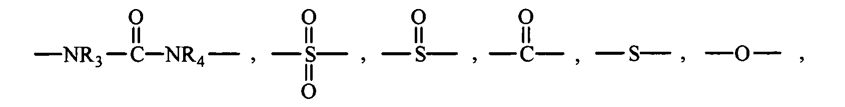 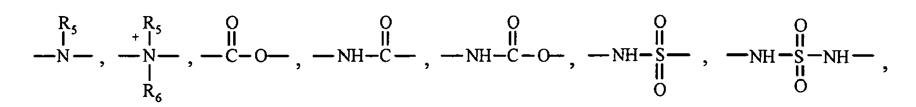 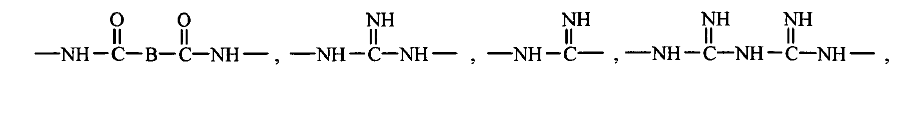
R₃, R₄, R₅ and R₆ each represent, independently of each other, H or a C₁-C₄ alkyl group;
B represents: 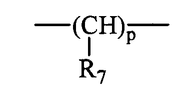
p represents an integer ranging from 0 to 6, and
R₇ represents H or OH, the symbols R₇ being identical or different when p is greater than 1,
or a salt thereof, for preventing the hair from breaking.

2. Use according to Claim 1, **characterized in that**
A represents: 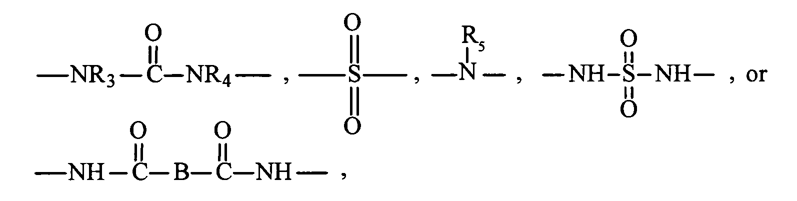
x = y = 0;
R₁ = R₂ = R₃ = R₄ = H;
R₅ = C₁-C₄ alkyl;
B represents 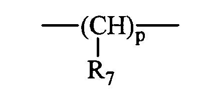 with p = 0 to 2 and R₇ = OH.

3. Use according to Claim 1 or 2, **characterized in that** the polyols are chosen from: 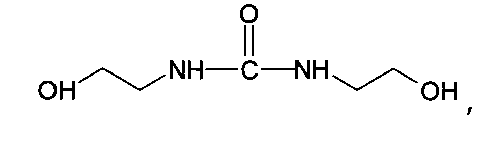 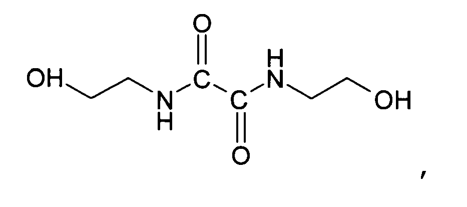 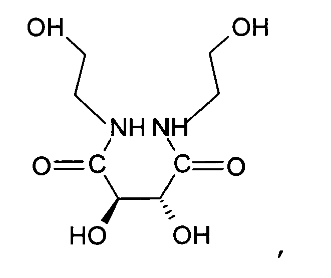 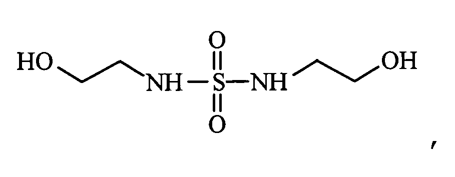 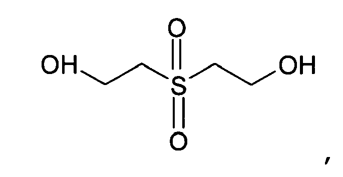 and

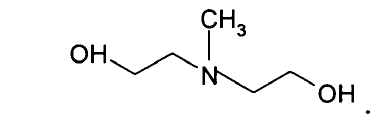

4. Use according to any one of the preceding claims, **characterized in that** the salts are chosen from C₂-C₈ alkyl hydrochlorides, sulfates and carboxylates.

5. Use according to Claim 4, **characterized in that** the hair is of African origin.

6. Cosmetic composition comprising, in a cosmetically acceptable medium, at least one polyol corresponding to formula (I') below: 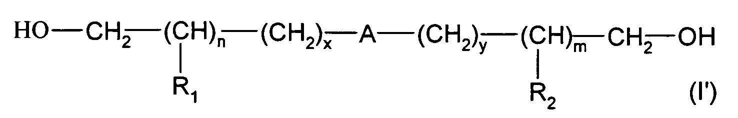 in which:
m and n each represent, independently of each other, an integer ranging from 0 to 6,
x and y each represent, independently of each other, 0 or 1,
R₁ and R₂ each represent, independently of each other, H, OH, -COOM, a halogen atom or a C₁-C₄ alkyl or -NR₁'R₂' group;
M represents a hydrogen atom, an alkali metal or alkaline-earth metal, or a C₁-C₄ alkyl group;
R₁' and R₂' each represent, independently of each other, H or a C₁-C₄ alkyl group;
A represents: 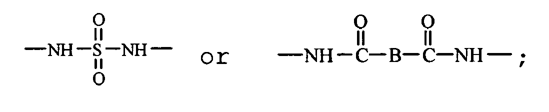
B represents: 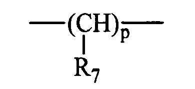
p represents an integer ranging from 1 to 6, and
R₇ represents H or OH, the symbols R₇ being identical or different when p is greater than 1, and at least one of the R₇ representing OH,
and/or a salt thereof.

7. Cosmetic composition according to Claim 6, **characterized in that**
A represents: 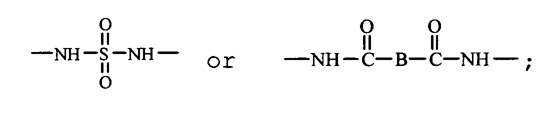
x = y = 0;
R₁ = R₂ = H;
R₅ = C₁-C₄ alkyl;
B represents 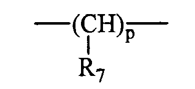 with p = 1 or and R₇ = OH.

8. Composition according to Claim 7, **characterized in that** the polyol is chosen from: 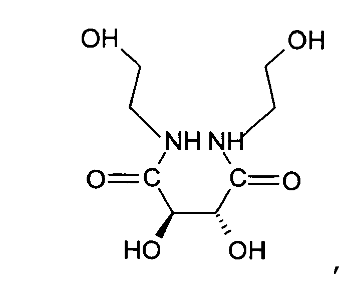 and

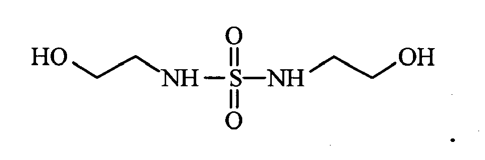

9. Composition according to any one of Claims 6 to 8, **characterized in that** the salts are chosen from C₂-C₈ alkyl hydrochlorides, sulfates and carboxylates.

10. Cosmetic composition according to any one of Claims 6 to 9, **characterized in that** the polyol(s) or salt(s) is (are) contained in an amount ranging from 0.05% to 35% by weight relative to the total weight of the composition.

11. Cosmetic composition according to Claim 10, **characterized in that** the polyol(s) or salt(s) is (are) contained in an amount ranging from 0.1% to 20% by weight relative to the total weight of the composition.

12. Composition according to any one of Claims 6 to 11, **characterized in that** the cosmetically acceptable medium consists solely of water or of at least one organic solvent, or alternatively of a mixture of water and of at least one organic solvent.

13. Composition according to Claim 12, **characterized in that** the organic solvent is a C₁-C₄ alcohol, a polyol, a polyol ether or an aromatic alcohol.

14. Composition according to Claim 13, **characterized in that** the organic solvent is ethanol, isopropanol, 2-butoxyethanol, propylene glycol, propylene glycol monomethyl ether, diethylene glycol monoethyl ether, diethylene glycol monomethyl ether, benzyl alcohol or phenoxyethanol.

15. Composition according to any one of Claims 6 to 14, **characterized in that** the composition comprises at least one additive chosen from anionic, cationic, nonionic, amphoteric or zwitterionic surfactants, anionic, cationic, nonionic, amphoteric or zwitterionic polymers, volatile or non-volatile, organomodified or non-organomodified silicones, mineral or organic thickeners, antioxidants, penetrating agents, sequestering agents, fragrances, buffers, dispersants, film-forming agents, preserving agents and opacifiers.

16. Use of the cosmetic composition according to any one of Claims 6 to 15, for preventing the hair from breaking.

17. Cosmetic hair treatment process comprising the application of a cosmetic composition according to any one of Claims 6 to 15 to dry or wet hair, followed by optional rinsing.

18. Cosmetic treatment process according to Claim 17, **characterized in that** the treatment temperature ranges from 10 to 60°C.

19. Cosmetic treatment process according to Claim 17 or 18, **characterized in that** the treatment lasts from 5 minutes to 5 days and preferably from 10 minutes to 3 days.

## Patentansprüche

1. Verwendung in der Kosmetik von Polyolen der Formel (I): 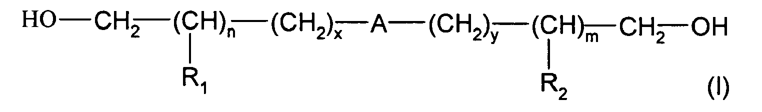 worin:
m und n jeweils unabhängig voneinander eine ganze Zahl von 0 bis 6 darstellen,
x und y jeweils unabhängig voneinander 0 oder 1 darstellen,
R₁ und R₂ jeweils unabhängig voneinander H, OH, -COOM, ein Halogenatom, eine C₁-C₄-Alkylgruppe oder -NR₁'R₂' darstellen,
M ein Wasserstoffatom, ein Alkali- oder Erdalkalimetall oder eine C₁-C₄-Alkylgruppe darstellt,
R₁' und R₂' jeweils unabhängig voneinander H oder eine C₁-C₄-Alkylgruppe darstellen,
A für Folgendes steht: 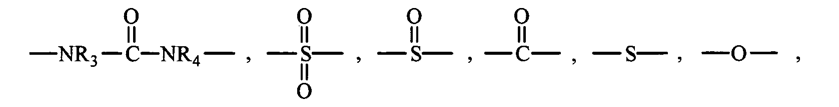 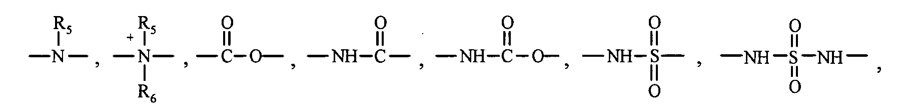 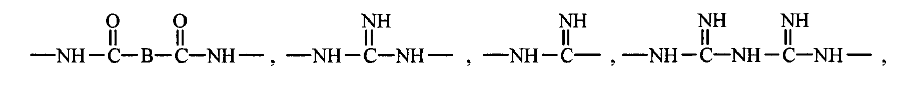
R₃, R₄, R₅ und R₆ jeweils unabhängig voneinander H oder eine C₁-C₄-Alkylgruppe darstellen,
B für Folgendes steht: 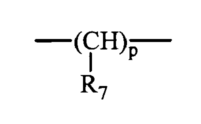
p eine ganze Zahl von 0 bis 6 darstellt und
R₇ H oder OH darstellt, wobei die Symbole R₇ gleich oder verschieden sind, wenn p größer als 1 ist,
oder eines ihrer Salze, um das Brechen von Haaren zu verhindern.

2. Verwendung nach Anspruch 1, **dadurch gekennzeichnet, dass** A für Folgendes steht: 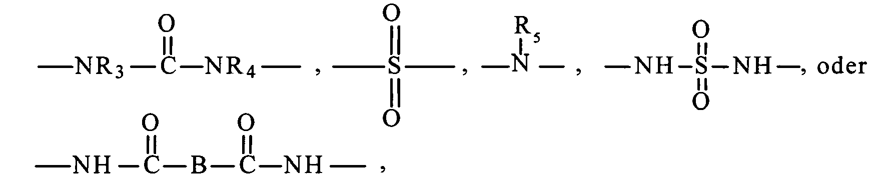
x = y = 0,
R₁ = R₂ = R₃ = R₄ = H,
R₅ = C₁-C₄-Alkyl
B für Folgendes steht: 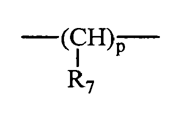 wobei p = 0 bis 2 und R₇ = OH.

3. Verwendung nach Anspruch 1 oder 2, **dadurch gekennzeichnet, dass** die Polyole ausgewählt sind aus: 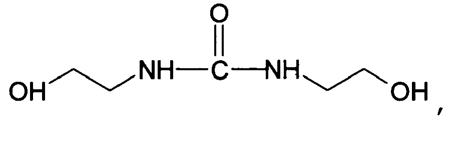 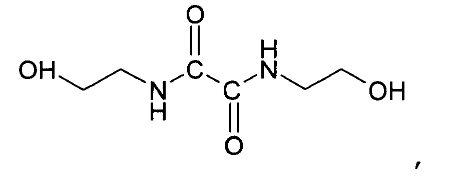 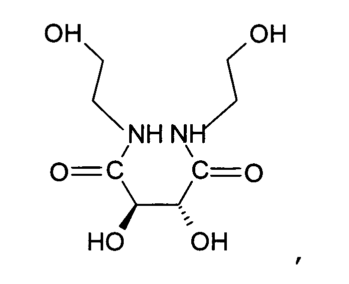 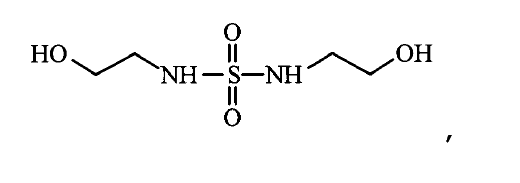 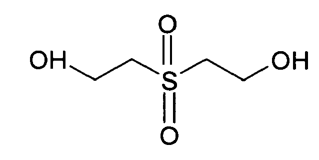 und

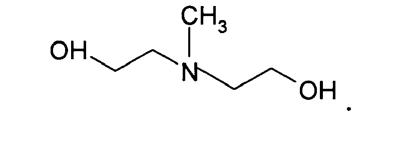

4. Verwendung nach einem der vorstehenden Ansprüche, **dadurch gekennzeichnet, dass** die Salze aus den C₂-C₈-Alkylhydrochloriden, -sulfaten und -carboxylaten ausgewählt sind.

5. Verwendung nach Anspruch 4, **dadurch gekennzeichnet, dass** die Haare afrikanischen Ursprungs sind.

6. Kosmetikzusammensetzung, umfassend in einem kosmetisch annehmbaren Medium mindestens ein Polyol der folgenden Formel (I'): 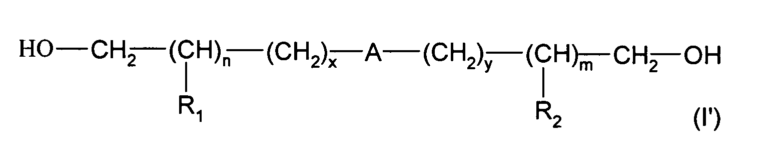 worin:
m und n jeweils unabhängig voneinander eine ganze Zahl von 0 bis 6 darstellen,
x und y jeweils unabhängig voneinander 0 oder 1 darstellen,
R₁ und R₂ jeweils unabhängig voneinander H, OH, -COOM, ein Halogenatom, eine C₁-C₄-Alkylgruppe oder -NR₁'R₂' darstellen,
M ein Wasserstoffatom, ein Alkali- oder Erdalkalimetall oder eine C₁-C₄-Alkylgruppe darstellt,
R₁' und R₂' jeweils unabhängig voneinander H oder eine C₁-C₄-Alkylgruppe darstellen,
A für Folgendes steht: 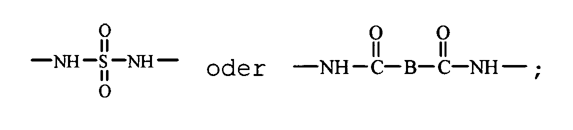
B für Folgendes steht: 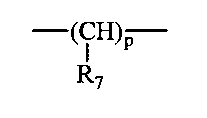
p eine ganze Zahl von 1 bis 6 darstellt und
R₇ H oder OH darstellt, wobei die Symbole R₇ gleich oder verschieden sind, wenn p größer als 1 ist, und mindestens einer der Reste R₇ für OH steht,
und/oder ein Salz davon.

7. Kosmetikzusammensetzung nach Anspruch 6, **dadurch gekennzeichnet, dass**
A für Folgendes steht: 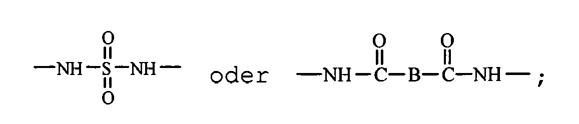
x = y = 0,
R₁ = R₂ = H,
B für Folgendes steht: 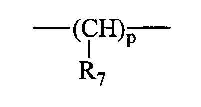 wobei p = 1 oder 2 und R₇ = OH.

8. Zusammensetzung nach Anspruch 7, **dadurch gekennzeichnet, dass** das Polyol ausgewählt ist aus: 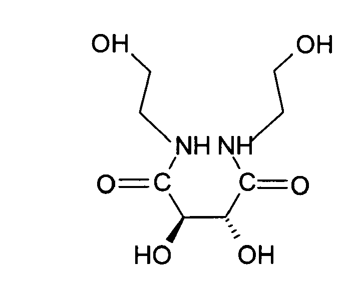 und

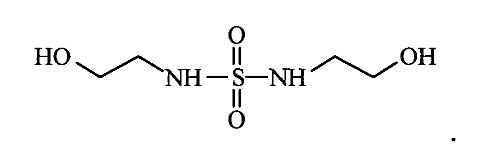

9. Zusammensetzung nach einem der Ansprüche 6 bis 8, **dadurch gekennzeichnet, dass** die Salze aus den C₂-C₈-Alkylhydrochloriden, -sulfaten und -carboxylaten ausgewählt sind.

10. Kosmetikzusammensetzung nach einem der Ansprüche 6 bis 9, **dadurch gekennzeichnet, dass** das oder die Polyol(e) oder Salz(e) in einer Menge von 0,05 bis 35 Gew.-%, bezogen auf das Gesamtgewicht der Zusammensetzung, enthalten sind.

11. Kosmetikzusammensetzung nach Anspruch 10, **dadurch gekennzeichnet, dass** das oder die Polyol(e) oder Salz(e) in einer Menge von 0,1 bis 20 Gew.-%, bezogen auf das Gesamtgewicht der Zusammensetzung, enthalten sind.

12. Zusammensetzung nach einem der Ansprüche 6 bis 11, **dadurch gekennzeichnet, dass** das kosmetisch annehmbare Medium einzig aus Wasser oder aus mindestens einem organischen Lösungsmittel oder auch aus einem Gemisch von Wasser und mindestens einem organischen Lösungsmittel besteht.

13. Zusammensetzung nach Anspruch 12, **dadurch gekennzeichnet, dass** das organische Lösungsmittel ein C₁-C₄-Alkohol, ein Polyol, ein Polyolether, ein aromatischer Alkohol ist.

14. Zusammensetzung nach Anspruch 13, **dadurch gekennzeichnet, dass** das organische Lösungsmittel Ethanol, Isopropanol, 2-Butoxyethanol, Propylenglykol, Propylenglykolmonomethylether, Diethylenglykolmonoethylether, Diethylenglykolmonomethylether, Benzylalkohol oder Phenoxyethanol ist.

15. Zusammensetzung nach einem der Ansprüche 6 bis 14, **dadurch gekennzeichnet, dass** die Zusammensetzung mindestens einen Zusatzstoff umfasst, ausgewählt aus anionischen, kationischen, nicht-ionischen, amphoteren oder zwitterionischen oberflächenaktiven Mitteln, anionischen, kationischen, nicht-ionischen, amphoteren oder zwitterionischen Polymeren, flüchtigen oder nichtflüchtigen, organomodifizierten oder nicht organomodifizierten Silikonen, anorganischen oder organischen Verdickungsmitteln, Antioxidantien, Durchdringungsmitteln, Sequestrierungsmitteln, Parfüms, Puffern, Dispergiermitteln, filmbildenden Mitteln, Konservierungsmitteln und Trübungsmitteln.

16. Verwendung der Kosmetikzusammensetzung nach einem der Ansprüche 6 bis 15 zur Verhinderung des Brechens von Haaren.

17. Verfahren zur kosmetischen Behandlung von Haaren, umfassend das Aufbringen einer Kosmetikzusammensetzung nach einem der Ansprüche 6 bis 15 auf die trockenen oder angefeuchteten Haare, gegebenenfalls gefolgt von Spülen.

18. Verfahren zur kosmetischen Behandlung nach Anspruch 17, **dadurch gekennzeichnet, dass** die Behandlungstemperatur von 10 bis 60°C reicht.

19. Verfahren zur kosmetischen Behandlung nach Anspruch 17 oder 18, **dadurch gekennzeichnet, dass** die Dauer der Behandlung von 5 Minuten bis 5 Tagen, vorzugsweise von 10 Minuten bis 3 Tagen, reicht.
